# EUROPEAN PATENT APPLICATION

(11) **EP 1 130 123 A2**
(43) Date of publication of application: **05.09.2001**
(21) Application number: 01301489.9
(22) Date of filing: 20.02.2001
(51) Int. Cl.: C12Q 1/68, C07K 14/71

(54) **Diagnostic method**

(30) Priority: 24.02.2000 GB 0004232
(71) Applicant: AstraZeneca AB, 151 85 Södertälje (SE)
(72) Inventor: Smith, John Craig, Macclesfield, Cheshire SK10 4TG (GB)
(74) Representative: Gainey, Laurence David Scott

(57) **Abstract**

This invention relates to novel sequence and polymorphisms in the human flt-1 gene. Eight specific polymorphisms are identified. The invention also relates to methods and materials for analysing allelic variation in the flt-1 gene and to the use of flt-1 polymorphism in the diagnosis and treatment of angiogenic diseases and cancer. Diseases associated with pathological angiogenesis include diabetic retinopathies, psoriasis, rheumatoid arthritis and endometriosis.

## Description

This invention relates to novel sequence and polymorphisms in the human flt-1 gene. The invention also relates to methods and materials for analysing allelic variation in the flt-1 gene and to the use of flt-1 polymorphism in the diagnosis and treatment of angiogenic diseases and cancer. Diseases associated with pathological angiogenesis include diabetic retinopathies, psoriasis, rheumatoid arthritis and endometriosis.

Flt-1 is one of the two receptors for vascular endothelial growth factor (VEGFR-1). The other being KDR (VEGFR-2). The flt-1 protein consists of an external domain containing seven immunoglobulin like domains, a transmembrane region and a cytoplasmic region containing a tyrosine kinase domain. In contrast to other members of the receptor tyrosine kinase family, the kinase domain of flt-1 is in two segments with an intervening sequence of ∼70 amino acids. The biology of the VEGF receptors has been reviewed (Neufeld et al., (1999) FASEB Journal. **13**:11-22; Zachary (1998) Experimental Nephrology. **6**:480-487) and the tyrosine phosphorylation sites have been identified (Ito et al., (1998) J. Biol. Chem. **273**:23410-23418).

It is thought that flt-1 may be important in regulating the tissue architecture in developing vasculature while the second VEGF receptor (KDR, VEGFR-2) mediates the mitogenic and angiogenic effects of VEGF in endothelial cells. Evidence to support this theory has come from knockout studies in mice (Fong et al., (1995) Nature. **376**:66-70).

VEGF and its receptors are over expressed in many tumour types and blocking of VEGF function inhibits angiogenesis and suppresses growth of tumours while over expression of VEGF enhances angiogenesis and tumour growth (Skobe et al., (1997) Nature Medicine **3**:1222-1227). Several studies have now shown that modulation of flt-1 activity can lead to anti-tumour activity. A small molecule inhibitor, SU5416, was originally developed against KDR but has been shown to be active against flt-1, the authors propose that inhibition of flt-1 may lead to interference with the formation of endothelial-matrix interactions (Fong et al., (1999) Cancer Research. **59**:99-106).

Alternative strategies to modulate flt-1 activity have included the use of ribozymes (Parry et al., (1999) Nucleic Acids Research. **27:**2569-2577), the synthesis of aptamers to inhibit binding of VEGF to its receptor (Ruckman et al., (1998) J Biol Chem. **273**:20556-20567) and the *in vivo* transfer of the flt-1 external domain (Kong et al., (1998) Human Gene Therapy. **9**:823-833). Chimeric toxins containing VEGF fused to the diptheria toxin have been used to target endothelial cells (Arora et al., (1999) Cancer Research. **59**:183-188).

The flt-1 cDNA (EMBL Accession Number X51602, 7680 bp) encodes a mature protein of 1338 amino acids. The structure of the murine flt-1 gene has been determined (Kondo *et al*., (1998) Gene **208**:297-305) and has been used to predict the intron/exon boundaries within the human gene. The promoter region of the human gene has been characterised (Ikeda *et al*., (1996) Growth Factors. **13:**151-162; Morishita *et al*., (1995) J Biol Chem **270**:27948-27953; EMBL Accession Number D64016,1745 bp). The flt-1 gene, which is organised into thirty exons, has been localised to chromosome 13q12 (Rosnet et al. (1993) Oncogene 8:73-179).

Unless otherwise indicated or apparent from the context, all exon positions herein relate to the positions indicated in EMBL Accession X51602, all promoter positions relate to the positions indicated in EMBL Accession No. 64016, and all intron sequences relate to one or other of SEQ ID Nos 1 - 5 disclosed herein.

SEQ ID No. 1 (1073 bp) represents exon 17 (positions 483 - 615 corresponding to positions 2605-2737 in EMBL Accession No. X51602) and adjacent intron sequences (positions 1-482 and 616-1073).

SEQ ID No. 2 (1480 bp) represents exon 21 (positions 438 - 594 corresponding to positions 3046-3202 in EMBL Accession No. X51602), exon 22 (positions 1025-1122 corresponding to positions 3203-3300 in EMBL Accession No. X51602) and intron sequences adjacent these exons (positions 1-437, 595-1024 and 1123-1480).

SEQ ID No. 3 (726 bp) represents exon 24 (positions 267-278 corresponding to positions 3424-3535 in EMBL Accession No. X51602) and adjacent intron sequences (positions 1-266 and 279-726).

SEQ ID No. 4 (1352 bp) represents exon 26 (positions 285-390 corresponding to positions 3636-3741 in EMBL Accession No. X51602), exon 27 (positions 652-794 corresponding to positions 3742-3884 in EMBL Accession No. X51602) and intron sequences adjacent these exons (positions 1-284, 391-651 and 795-1352).

SEQ ID No. 5 (1256 bp) represents exon 28 (positions 580-664 corresponding to positions 3885-3969 in EMBL Accession No. X51602) and adjacent intron sequences (positions 1-579 and 665-1256).

The novel intron sequence, or parts thereof, can be used, *inter alia,* as hybridisation probes to identify clones harbouring the flt-1 gene, for use in genetic linkage studies or for design and use as amplification primers suitable, for example, to amplify some or all of the flt-1 gene using an amplification reaction such as the PCR.

Polymorphism refers to the occurrence of two or more genetically determined alternative alleles or sequences within a population. A polymorphic marker is the site at which divergence occurs. Preferably markers have at least two alleles, each occurring at frequency of greater than 1%, and more preferably at least 10%, 15%, 20%, 30% or more of a selected population.

Single nucleotide polymorphisms (SNP) are generally, as the name implies, single nucleotide or point variations that exist in the nucleic acid sequence of some members of a species. Such polymorphism variation within the species are generally regarded to be the result of spontaneous mutation throughout evolution. The mutated and normal sequences coexist within the species' population sometimes in a stable or quasi-stable equilibrium. At other times the mutation may confer some selective advantage to the species and with time may be incorporated into the genomes of all members of the species.

Some SNPs occur in the protein coding sequences, in which case, one of the polymorphic protein forms may possess a different amino acid which may give rise to the expression of a variant protein and, potentially, a genetic disease. Polymorphisms may also affect mRNA synthesis, maturation, transportation and stability. Polymorphisms which do not result in amino acid changes (silent polymorphisms) or which do not alter any known consensus sequences may nevertheless have a biological effect, for example by altering mRNA folding, stability, splicing, transcription rate, translation rate, or fidelity. Recently, it has been reported that even polymorphisms that do not result in an amino acid change can cause different structural folds of mRNA with potentially different biological functions (Shen *et al*., (1999) Proc Natl Acad Sci USA **96**:7871-7876). Thus, changes that occur outside of the coding region, i.e. intron sequences, promoter regions etc may affect the transcription and/or message stability of the sequences and thus affect the level of the protein (receptor) in cells.

The use of knowledge of polymorphisms to help identify patients most suited to therapy with particular pharmaceutical agents is often termed "pharmacogenetics". Pharmacogenetics can also be used in pharmaceutical research to assist the drug selection process. Polymorphisms are used in mapping the human genome and to elucidate the genetic component of diseases. The reader is directed to the following references for background details on pharmacogenetics and other uses of polymorphism detection: Linder *et al*. (1997), Clinical Chemistry, **43**:254; Marshall (1997), Nature Biotechnology, **15**:1249; International Patent Application WO 97/40462, Spectra Biomedical; and Schafer *et al*, (1998), Nature Biotechnology, **16**:33.

A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each SNP and n is the number of SNPs. One approach to identifying mutations or polymorphisms which are correlated with clinical response is to carry out an association study using all the haplotypes that can be identified in the population of interest. The frequency of each haplotype is limited by the frequency of its rarest allele, so that SNPs with low frequency alleles are particularly useful as markers of low frequency haplotypes. As particular mutations or polymorphisms associated with certain clinical features, such as adverse or abnormal events, are likely to be of low frequency within the population, low frequency SNPs may be particularly useful in identifying these mutations (for examples see: Linkage disequilibrium at the cystathionine beta synthase (CBS) locus and the association between genetic variation at the CBS locus and plasma levels of homocysteine. *Ann Hum Genet* (1998) **62**:481-90, De Stefano V, Dekou V, Nicaud V, Chasse JF, London J, Stansbie D, Humphries SE, and Gudnason V; and Variation at the von willebrand factor (vWF) gene locus is associated with plasma vWF:Ag levels: identification of three novel single nucleotide polymorphisms in the vWF gene promoter. *Blood* (1999) **93**:4277-83, Keightley AM, Lam YM, Brady JN, Cameron CL, Lillicrap D).

Clinical trials have shown that patient response to drugs is heterogeneous. Thus there is a need for improved approaches to pharmaceutical agent design and therapy.

Point mutations in polypeptides will be referred to as follows: natural amino acid (using 1 or 3 letter nomenclature), position, new amino acid. For (a hypothetical) example, "D25K" or "Asp25Lys" means that at position 25 an aspartic acid (D) has been changed to lysine (K). Multiple mutations in one polypeptide will be shown between square brackets with individual mutations separated by commas.

The present invention is based on the discovery of nine novel single nucleotide polymorphisms as well as novel intronic sequence of the flt-1 gene. Relative to EMBL Accession No. X51602 the three novel coding sequence polymorphisms are located at nucleotide position: 1953, 3453 and 3888. Relative to EMBL Accession No. D64016 the four novel promoter sequence polymorphisms are located at nucleotide position: 519, 786, 1422 and 1429. Relative to SEQ ID No.3, the intron 24 polymorphism is located at position 454. Relative to SEQ ID No.5, the intron 28 polymorphism is located at position 696.

For the avoidance of doubt the location of each of the polymorphisms (emboldened; published allele (if published) illustrated first) and sequence immediately flanking each polymorphism site is as follows:
Numbering according to EMBL Accession X51602
a) Position 1953 (codon 568 polymorphism) 
GGAAAAAATGCCGACG/AGAAGGAGAGGACCTG    (SEQ ID No. 6)
1938                                                                                    1968
b) Position 3453 (codon 1068 polymorphism) 
GAAATGGATGGCTCCC/TGAATCTATCTTTGAC (SEQ ID No. 7)
3438 3468
c) Position 3888 (codon 1213 polymorphism) 
TGATGATGTCAGATAT/CGTAAATGCTTTCAAG (SEQ ID No. 8)
3873 3903
 Numbering according to EMBL Accession D64016
d) Position 519 (promoter polymorphism) 
AAAAAGACACGGACAC/TGCTCCCCTGGGACCT (SEQ ID No. 9)
504 534
e) Position 786 (promoter polymorphism) 
GATCGGACTTTCCGCC/TCCTAGGGCCAGGCGG (SEQ ID No.10)
771 801
f) Position 1422 (promoter polymorphism) 
GACGGACTCTGGCGGC/TCGGGTCTTTGGCCGC (SEQ ID No. 11)
1407 1437
g) Position 1429 (promoter polymorphism) 
TCTGGCGGCCGGGTCG/TTTGGCCGCGGGAGC (SEQ ID No. 12)
1414 1444
 Numbering according to Seq ID 3 (intron 24)
h) Intron 24 position 454 
GAATGTCCTTTGGTTG/AGACAGCCTTTAGATT (SEQ ID No. 13)
439 469
 Numbering according to Seq ID No 5 (intron 28)
i) Intron 28 position 696

AGGTACCTAGTGCACT/CCCGATAGACCCCTTC (SEQ ID No. 14)
681 711

According to one aspect of the present invention there is provided a method for the diagnosis of one or more single nucleotide polymorphism(s) in flt-1 gene in a human, which method comprises determining the sequence of the nucleic acid of the human at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), and determining the status of the human by reference to polymorphism in the flt-1 gene.

The term human includes both a human having or suspected of having a flt-1 ligand-mediated disease and an asymptomatic human who may be tested for predisposition or susceptibility to such disease. At each position the human may be homozygous for an allele or the human may be a heterozygote.

The term 'flt-1-ligand mediated disease' means any disease which results from pathological changes in the level or activity of the flt-1 ligand (VEGF).

The term 'flt-1 drug' means any drug which changes the level of an flt-1-ligand mediated response or changes the biological activity of flt-1 (VEGFR-1). For example the drug may be an agonist or an antagonist of a natural ligand for flt-1. A drug which inhibits the activity of the flt-1 (VEGFR-1) is preferred.

As defined herein, the flt-1 gene includes exon coding sequence, intron sequences intervening the exon sequences and, 3' and 5' untranslated region (3' UTR and 5' UTR) sequences, including the promoter element of the flt-1 gene.

In one embodiment of the invention preferably the method for diagnosis described herein is one in which the single nucleotide polymorphism at position 1953 (according to the position in EMBL accession number X51602) is the presence of G and/or A.

In another embodiment of the invention preferably the method for diagnosis described herein is one in which the single nucleotide polymorphism at position 3453 (according to the position in EMBL accession number X51602) is the presence of C and/or T.

In another embodiment of the invention preferably the method for diagnosis described herein is one in which the single nucleotide polymorphism at position 3888 (according to the position in EMBL accession number X51602) is the presence of T and/or C.

In another embodiment of the invention preferably the method for diagnosis described herein is one in which the single nucleotide polymorphism at position 519 (according to the position in EMBL accession number D64016) is the presence of C and/or T.

In another embodiment of the invention preferably the method for diagnosis described herein is one in which the single nucleotide polymorphism at position 786 (according to the position in EMBL accession number D64016) is the presence of C and/or T.

In another embodiment of the invention preferably the method for diagnosis described herein is one in which the single nucleotide polymorphism at position 1422 (according to the position in EMBL accession number D64016) is the presence of C and/or T.

In another embodiment of the invention preferably the method for diagnosis described herein is one in which the single nucleotide polymorphism at position 1429 (according to the position in EMBL accession number D64016) is the presence of G and/or T.

In another embodiment of the invention preferably the method for diagnosis described herein is one in which the single nucleotide polymorphism at position 454 (according to the position in SEQ ID No. 3) is the presence of G and/or A.

In another embodiment of the invention preferably the method for diagnosis described herein is one in which the single nucleotide polymorphism at position 696 (according to the position in SEQ ID No. 5) is the presence of T and/or C.

The method for diagnosis is preferably one in which the sequence is determined by a method selected from amplification refractory mutation system (ARMS™-allele specific amplification), allele specific hybridisation (ASH), oligonucleotide ligation assay (OLA) and restriction fragment length polymorphism (RFLP).

In another aspect of the invention there is provided a method of analysing a nucleic acid, comprising: obtaining a nucleic acid from an individual; and determining the base occupying any one of the following polymorphic sites: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5).

In another aspect of the invention we provide a method for the diagnosis of flt-1 ligand-mediated disease, which method comprises:
i) obtaining sample nucleic acid from an individual;
ii) detecting the presence or absence of a variant nucleotide at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), in the flt-1 gene; and,
iii) determining the status of the individual by reference to polymorphism in the flt-1 gene.

Allelic variation at position 1953 (according to EMBL sequence X51602) consists of a single base substitution from G (the published base), for example to A. Allelic variation at position 3453 (according to EMBL sequence X51602) consists of a single base substitution from C (the published base), for example to T. Allelic variation at position 3888 (according to EMBL sequence X51602) consists of a single base substitution from T (the published base), for example to C. Allelic variation at position 519 (according to EMBL sequence D64016), consists of a single base substitution from C (the published base), for example to T. Allelic variation at position 786 (according to EMBL sequence D64016), consists of a single base substitution from C (the published base), for example to T. Allelic variation at position 1422 (according to EMBL sequence D64016), consists of a single base substitution from C (the published base), for example to T. Allelic variation at position 1429 (according to EMBL sequence D64016), consists of a single base substitution from G (the published base), for example to T. Allelic variation at position 454 (according to SEQ ID No. 3) consists of a single base substitution from C to G, for example. Allelic variation at position 696 (according to SEQ ID No. 5) consists of a single base substitution from T to C, for example.

The invention resides in the identification of the existence of different alleles at particular loci. The status of the individual may be determined by reference to allelic variation at one, two, three, four, five, six, seven or all eight positions optionally in combination with any other polymorphism in the gene that is (or becomes) known.

The test sample of nucleic acid is conveniently a sample of blood, bronchoalveolar lavage fluid, sputum, urine or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before use in the analysis of sequence variation.

It will be apparent to the person skilled in the art that there are a large number of analytical procedures which may be used to detect the presence or absence of one or more of the polymorphisms identified herein. In general, the detection of allelic variation requires a mutation discrimination technique, optionally an amplification reaction and a signal generation system. Table 1 lists a number of mutation detection techniques, some based on the PCR. These may be used in combination with a number of signal generation systems, a selection of which is listed in Table 2. Further amplification techniques are listed in Table 3. Many current methods for the detection of allelic variation are reviewed by Nollau *et al*., Clin. Chem. **43,** 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

| **Abbreviations:** | |
|---|---|
| ALEX™ | Amplification refractory mutation system linear extension |
| APEX | Arrayed primer extension |
| ARMS™ | Amplification refractory mutation system |
| ASH | Allele specific hybridisation |
| b-DNA | Branched DNA |
| CMC | Chemical mismatch cleavage |
| bp | base pair |
| COPS | Competitive oligonucleotide priming system |
| DGGE | Denaturing gradient gel electrophoresis |
| FRET | Fluorescence resonance energy transfer |
| LCR | Ligase chain reaction |
| MASDA | Multiple allele specific diagnostic assay |
| NASBA | Nucleic acid sequence based amplification |
| flt-1 | VEGF receptor-1 |
| OLA | Oligonucleotide ligation assay |
| PCR | Polymerase chain reaction |
| PTT | Protein truncation test |
| RFLP | Restriction fragment length polymorphism |
| SERRS | Surface enhanced raman resonance spectroscopy |
| SDA | Strand displacement amplification |
| SNP | Single nucleotide polymorphism |
| SSCP | Single-strand conformation polymorphism analysis |
| SSR | Self sustained replication |
| TGGE | Temperature gradient gel electrophoresis |

### Table 1 - Mutation Detection Techniques

**General:** DNA sequencing, Sequencing by hybridisation

**Scanning:** PTT^{*}, SSCP, DGGE, TGGE, Cleavase, Heteroduplex analysis, CMC, Enzymatic mismatch cleavage

* Note: not useful for detection of promoter polymorphisms.

### Hybridisation Based

Solid phase hybridisation: Dot blots, MASDA, Reverse dot blots, Oligonucleotide arrays (DNA Chips)

Solution phase hybridisation: Taqman™ - US-5210015 & US-5487972 (Hoffmann-La Roche), Molecular Beacons - Tyagi *et al* (1996), Nature Biotechnology, **14**, 303; WO 95/13399 (Public Health Inst., New York), ASH
**Extension Based:** ARMS™-allele specific amplification (as described in European patent No. EP-B-332435 and US patent No. 5,595,890), ALEX™ - European Patent No. EP 332435 B1 (Zeneca Limited), COPS - Gibbs *et al* (1989), Nucleic Acids Research, **17,**2347. **Incorporation Based:** Mini-sequencing, APEX
**Restriction Enzyme Based:** RFLP, Restriction site generating PCR
**Ligation Based:** OLA- Nickerson et al. (1990) P.N.A.S. 87:8923-8927.
**Other:** Invader assay

### Table 2 - Signal Generation or Detection Systems

**Fluorescence:** FRET, Fluorescence quenching, Fluorescence polarisation - United Kingdom Patent No. 2228998 (Zeneca Limited)

**Other:** Chemiluminescence, Electrochemiluminescence, Raman, Radioactivity, Colorimetric, Hybridisation protection assay, Mass spectrometry, SERRS - WO 97/05280 (University of Strathclyde).

### Table 3 - Further Amplification Methods

### SSR, NASBA, LCR, SDA, b-DNA

Preferred mutation detection techniques include ARMS™-allele specific amplification, ALEX™, COPS, Taqman, Molecular Beacons, RFLP, OLA, restriction site based PCR and FRET techniques.

Particularly preferred methods include ARMS™-allele specific amplification, OLA and RFLP based methods. The allele specific amplification technique known in the art as ARMS™ is an especially preferred method.

ARMS™-allele specific amplification (described in European patent No. EP-B-332435, US patent No. 5,595,890 and Newton et al. (Nucleic Acids Research, Vol. 17, p.2503; 1989)), relies on the complementarity of the 3' terminal nucleotide of the primer and its template. The 3' terminal nucleotide of the primer being either complementary or non-complementary to the specific mutation, allele or polymorphism to be detected. There is a selective advantage for primer extension from the primer whose 3' terminal nucleotide complements the base mutation, allele or polymorphism. Those primers which have a 3' terminal mismatch with the template sequence severely inhibit or prevent enzymatic primer extension. Polymerase chain reaction or unidirectional primer extension reactions therefore result in product amplification when the 3' terminal nucleotide of the primer complements that of the template, but not, or at least not efficiently, when the 3' terminal nucleotide does not complement that of the template.

Therapeutic opportunities for VEGF receptor antagonists exist for angiogenic and cancer diseases. An example of a known inhibitor of flt-1 is SU5416 *(supra).*

In a further aspect, the diagnostic methods of the invention are used to assess the efficacy of therapeutic compounds in the treatment of angiogenic diseases, such as diabetic retinopathies, psoriasis, rheumatoid arthritis and endometriosis, and cancer.

The polymorphisms identified in the present invention that occur in intron regions or in the promoter region are not expected to alter the amino acid sequence of the flt-1 receptor, but may affect the transcription and/or message stability of the sequences and thus affect the level of the receptors in cells.

Assays, for example reporter-based assays, may be devised to detect whether one or more of the above polymorphisms affect transcription levels and/or message stability.

Individuals who carry particular allelic variants of the flt-1 gene, especially those within the promoter element, may therefore exhibit differences in receptor levels under different physiological conditions and will display altered abilities to react to different diseases. In addition, differences in receptor level arising as a result of allelic variation may have a direct effect on the response of an individual to drug therapy. Flt-1 polymorphism may therefore have the greatest effect on the efficacy of drugs designed to modulate the activity of the flt-1. However, the polymorphisms may also affect the response to agents acting on other biochemical pathways regulated by a flt-1 ligand. The diagnostic methods of the invention may therefore be useful both to predict the clinical response to such agents and to determine therapeutic dose.

In a further aspect, the diagnostic methods of the invention, are used to assess the predisposition and/or susceptibility of an individual to diseases mediated by an flt-1 ligand.

Flt-1 gene polymorphism may be particularly relevant in the development of diseases modulated by an flt-1 ligand. The present invention may be used to recognise individuals who are particularly at risk from developing these conditions.

In a further aspect, the diagnostic methods of the invention are used in the development of new drug therapies which selectively target one or more allelic variants of the flt-1 gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes. For example the polymorphism at position 3888 (numbering according to EMBL sequence X51602) that alters the third base of codon 1213 can be detected by digestion with the restriction enzyme Sna 1B, as polymorphism at this position creates a Sna IB recognition sequence (TA**C**GTA).

Engineered sites include those wherein the primer sequences employed to amplify the target sequence participates along with the nucleotide polymorphism to create a restriction site For example, the polymorphism at position 519 (numbering according to EMBL sequence D64016) can be detected by diagnostic engineered RFLP digestion with the restriction enzyme Sph 1, since modification of position 516 creates a potential Sph 1 I recognition sequence (GCATGC). Polymorphism at position 519 will modify the recognition sequence (GCAC/TGC).

The person of ordinary skill will be able to design and implement diagnostic procedures based on the detection of restriction fragment length polymorphism due to the loss or gain of one or more of the sites.

According to another aspect of the present invention there is provided a nucleic acid comprising any one of the following polymorphisms:
the nucleic acid disclosed in EMBL Accession Number X51602 with A at position 1953 according to the nucleotide positioning therein;
the nucleic acid sequence disclosed in EMBL Accession Number X51602 with T at position 3453 according to the nucleotide positioning therein;
the nucleic acid sequence disclosed in EMBL Accession Number X51602 with C at position 3888 according to the nucleotide positioning therein;
the nucleic acid sequence disclosed in EMBL Accession Number D64016 with T at position 519 according to the nucleotide positioning therein;
the nucleic acid sequence disclosed in EMBL Accession Number D64016 with T at position 786 according to the nucleotide positioning therein;
the nucleic acid sequence disclosed in EMBL Accession Number D64016 with T at position 1422 according to the nucleotide positioning therein;
the nucleic acid sequence disclosed in EMBL Accession Number D64016 with T at position 1429 according to the nucleotide positioning therein;
the nucleic acid sequence disclosed in SEQ ID No. 3 with G at position 454 according to the nucleotide positioning therein;
the nucleic acid sequence disclosed in SEQ ID No. 3 with A at position 454 according to the nucleotide positioning therein;
the nucleic acid sequence disclosed in SEQ ID No. 5 with T at position 696 according to the nucleotide positioning therein;
the nucleic acid sequence disclosed in SEQ ID No. 5 with C at position 696 according to the nucleotide positioning therein;
or a complementary strand thereof or a fragment thereof of at least 17 bases comprising at least one of the polymorphisms.

According to another aspect of the present invention there is provided an isolated nucleic acid comprising at least 17 consecutive bases of flt-1 gene said nucleic acid comprising one or more of the following polymorphic alleles: A at position 1953 (according to X51602), T at position 3453 (according to X51602), C at position 3888 (according to X51602), T at position 519 (according to D64016), T at position 786 (according to D64016), T at position 1422 (according to D64016), T at position 1429 (according to D64016), A at position 454 (according to SEQ ID No. 3) and C at position 696 (according to SEQ ID No. 5), or a complementary strand thereof.

Fragments are at least 17 bases more preferably at least 20 bases, more preferably at least 30 bases.

The invention further provides nucleotide primers which detect the flt-1 gene polymorphisms of the invention. Such primers can be of any length, for example between 8 and 100 nucleotides in length, but will preferably be between 12 and 50 nucleotides in length, more preferable between 17 and 30 nucleotides in length.

According to another aspect of the present there is provided an allele specific primer capable of detecting an flt-1 gene polymorphism at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5).

An allele specific primer is used, generally together with a constant primer, in an amplification reaction such as PCR, which provides the discrimination between alleles through selective amplification of one allele at a particular sequence position e.g. as used for ARMS™ allele specific amplification assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer. Often the nucleotide at the -2 and/or -3 position (relative to the 3' terminus) is mismatched in order to optimise differential primer binding and preferential extension from the correct allele discriminatory primer only

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a flt-1 gene polymorphism of the invention.

According to another aspect of the present invention there is provided an allele-specific oligonucleotide probe capable of detecting an flt-1 gene polymorphism at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), in the flt-1 gene.

The allele-specific oligonucleotide probe is preferably 17- 50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. Suitable oligonucleotide probes might be those consisting of or comprising the sequences depicted in SEQ ID Nos. 6 - 14 possessing one or other of the central allelic base differences (emboldened), or sequences complementary thereto. The probes or primers of the invention may carry one or more labels to facilitate detection, such as in Molecular Beacons.

According to another aspect of the present invention there is provided a diagnostic kit comprising one or more allele-specific primers of the invention and/or one or more allele-specific oligonucleotide probe of the invention.

The diagnostic kits may comprise appropriate packaging and instructions for use in the methods of the invention. Such kits may further comprise appropriate buffer(s) and polymerase(s) such as thermostable polymerases, for example taq polymerase. Such kits may also comprise companion primers and/or control primers or probes. A companion primer is one that is part of the pair of primers used to perform PCR. Such primer usually complements the template strand precisely.

In another aspect of the invention, the single nucleotide polymorphisms of this invention may be used as genetic markers for this region in linkage studies. This particularly applies to the polymorphisms at positions 3453, 3888 (both according to the position in EMBL Accession No. X51602), position 1429 (according to the position in EMBL accession number D64016), position 454 (according to the position in SEQ ID No. 3) and position 696 (according to the position in SEQ ID No. 5) because of their relatively high frequency. Those polymorphisms that occur relatively infrequently are useful as markers of low frequency haplotypes.

According to another aspect of the present invention there is provided a method of treating a human in need of treatment with an flt-1 ligand antagonist drug in which the method comprises:
i) diagnosis of a single nucleotide polymorphism in flt-1 gene in the human, which diagnosis comprises determining the sequence of the nucleic acid at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5);
ii) determining the status of the human by reference to polymorphism in the flt-1 gene; and
ii) administering an effective amount of an flt-1 ligand antagonist drug.

Preferably determination of the status of the human is clinically useful. Examples of clinical usefulness include deciding which flt-1 ligand antagonist drug or drugs to administer and/or in deciding on the effective amount of the drug or drugs.

According to another aspect of the present invention there is provided use of an flt-1 ligand antagonist drug in the preparation of a medicament for treating a VEGF-mediated disease in a human diagnosed as having a single nucleotide polymorphism at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), in the flt-1 gene.

According to another aspect of the present invention there is provided a pharmaceutical pack comprising an flt-1 ligand antagonist drug and instructions for administration of the drug to humans diagnostically tested for a single nucleotide polymorphism at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), in the flt-1 gene.

According to another aspect of the invention there is provided an isolated nucleic acid sequence comprising the sequence selected from the group consisting of:
(i) the nucleotide sequence from positions 1-482 of SEQ ID No. 1;
(ii) the nucleotide sequence from positions 616-1073 of SEQ ID No. 1;
(iii) the nucleotide sequence from positions 1-437 of SEQ ID No. 2;
(iv) the nucleotide sequence from positions 595-1024 of SEQ ID No. 2;
(v) the nucleotide sequence from positions 1123-1480 of SEQ ID No. 2;
(vi) the nucleotide sequence from positions 1-266 of SEQ ID No. 3;
(vii) the nucleotide sequence from positions 279-726 of SEQ ID No. 3;
(viii) the nucleotide sequence from positions 1-284 of SEQ ID No. 4;
(ix) the nucleotide sequence from positions 391-651 of SEQ ID No. 4;
(x) the nucleotide sequence from positions 795-1352 of SEQ ID No. 4;
(xi) the nucleotide sequence from positions 1-579 of SEQ ID No. 5;
(xii) the nucleotide sequence from positions 665-1256 of SEQ ID No. 5;
(xiii) a nucleotide sequence having at least 80%, preferably at least 90%, sequence identity to a sequences (i) - (xii);
(xiv) an isolated fragment of (i) - (xiii); and
(xv) a nucleotide sequence fully complementary to (i) - (xiv).

In the above, group (xiii) relates to variants of the polynucleotide depicted in groups (i) - (xii). The variant of the polynucleotide may be a naturally occurring allelic variant, from the same species or a different species, or a non-naturally occurring allelic variant. As known in the art an allelic variant is an alternate form of a polynucleotide sequence which may have a deletion, addition or substitution of one or more nucleotides.

Sequence identity can be assessed by best-fit computer alignment analysis using suitable software such as Blast, Blast2, FastA, Fasta3 and PILEUP. Preferred software for use in assessing the percent identity, i.e how two polynucleotide sequences line up is PILEUP. Identity refers to direct matches. In the context of the present invention, two polynucleotide sequences with 90% identity have 90% of the nucleotides being identical and in a like position when aligned optimally allowing for up to 10, preferably up to 5 gaps. The present invention particularly relates to polynucleotides which hybridise to one or other of the polynucleotide sequences (i) - (xv), under stringent conditions. As used herein, stringent conditions are those conditions which enable sequences that possess at least 80%, preferably at least 90%, more preferably at least 95% and more preferably at least 98% sequence identity to hybridise together. Thus, nucleic acids which can hybridise to one or other of the nucleic acids of (I) - (xv), include nucleic acids which have at least 80%, preferably at least 90%, more preferably at least 95%, even more preferably at least 98% sequence identity and most preferably 100%, over at least a portion (at least 20, preferably 30 or more consecutive nucleotides) of the polynucleotide sequence of (i) - (xv) above.

As well as the novel intron sequences depicted in SEQ ID Nos. 1 - 5, smaller nucleic acid fragments thereof useful for example as oligonucleotide primers to amplify the flt-1 gene sequences or identify SNPs using any of the well known amplification systems such as the polymerase chain reaction (PCR), or fragments that can be used as diagnostic probes to identify corresponding nucleic acid sequences are also part of this invention. The invention thus includes polynucleotides of shorter length than the novel intron flt-1 sequences depicted in SEQ ID Nos.1 - 5 that are capable of specifically hybridising to the sequences depicted herein. Such polynucleotides may be at least 17 nucleotides in length, preferably at least 20, more preferably at least 30 nucleotides in length and may be of any size up to and including or indeed, comprising the complete intron sequences depicted in SEQ ID Nos. 1 - 5.

An example of a suitable hybridisation solution when a nucleic acid is immobilised on a nylon membrane and the probe nucleic acid is greater than 300 bases or base pairs, say 500 bp, is: 6 x SSC (saline sodium citrate), 0.5% SDS (sodium dodecyl sulphate), 100µg/ml denatured, sonicated salmon sperm DNA. An example of a suitable hybridisation solution when a nucleic acid is immobilised on a nylon membrane and the probe is an oligonucleotide of between 12 and 50 bases is: 3M trimethylammonium chloride (TMACI), 0.01M sodium phosphate (pH 6.8), 1mM EDTA (pH 7.6), 0.5% SDS,100µg/ml denatured, sonicated salmon sperm DNA and 0.1% dried skimmed milk. The hybridisation can be performed at 68°C for at least 1 hour and the filters then washed at 68°C in 1 x SSC, or for higher stringency, 0.1 x SSC/0.1% SDS. Hybridisation techniques are well advanced in the art. The person skilled in the art will be able to adapt the hybridisation conditions to ensure hybridisation of sequences with 80%, 90% or more identity.

A fragment can be any part of the full length sequence and may be single or double stranded or may comprise both single and double stranded regions. In a preferred embodiment, a fragment is a restriction enzyme fragment.

The nucleic acid sequences of the invention, particularly those relating to and identifying the single nucleotide polymorphisms identified herein represent a valuable information source with which to identify further sequences of similar identity and characterise individuals in terms of, for example, their identity, haplotype and other sub-groupings, such as susceptibility to treatment with particular drugs. These approaches are most easily facilitated by storing the sequence information in a computer readable medium and then using the information in standard macromolecular structure programs or to search sequence databases using state of the art searching tools such as GCG (Genetics Computer Group), BlastX BlastP, BlastN, FASTA (refer to Altschul et al. J. Mol. Biol. 215:403-410, 1990). Thus, the nucleic acid sequences of the invention are particularly useful as components in databases useful for sequence identity, genome mapping, pharmacogenetics and other search analyses. Generally, the sequence information relating to the nucleic acid sequences and polymorphisms of the invention may be reduced to, converted into or stored in a tangible medium, such as a computer disk, preferably in a computer readable form. For example, chromatographic scan data or peak data, photographic scan or peak data, mass spectrographic data, sequence gel (or other) data.

The invention provides a computer readable medium having stored thereon one or more nucleic acid sequences of the invention. For example, a computer readable medium is provided comprising and having stored thereon a member selected from the group consisting of: a nucleic acid comprising the sequence of a nucleic acid of the invention, a nucleic acid consisting of a nucleic acid of the invention, a nucleic acid which comprises part of a nucleic acid of the invention, which part includes at least one of the polymorphisms of the invention, a set of nucleic acid sequences wherein the set includes at least one nucleic acid sequence of the invention, a data set comprising or consisting of a nucleic acid sequence of the invention or a part thereof comprising at least one of the polymorphisms identified herein. The computer readable medium can be any composition of matter used to store information or data, including, for example, floppy disks, tapes, chips, compact disks, digital disks, video disks, punch cards and hard drives.

In another aspect of the invention there is provided a computer readable medium having stored thereon a nucleic acid sequence comprising at least 20 consecutive bases of the flt-1 gene sequence, which sequence includes at least one of the polymorphisms at positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5).

In another aspect of the invention there is provided a computer readable medium having stored thereon a nucleic acid comprising any of the intron sequences disclosed in any of SEQ ID Nos. 1 - 5.

A computer based method is also provided for performing sequence identification, said method comprising the steps of providing a nucleic acid sequence comprising a polymorphism of the invention in a computer readable medium; and comparing said polymorphism containing nucleic acid sequence to at least one other nucleic acid or polypeptide sequence to identify identity (homology), i.e. screen for the presence of a polymorphism. Such a method is particularly useful in pharmacogenetic studies and in genome mapping studies.

In another aspect of the invention there is provided a method for performing sequence identification, said method comprising the steps of providing a nucleic acid sequence comprising at least 20 consecutive bases of the flt-1 gene sequence, which sequence includes at least one of the polymorphisms at positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5) in a computer readable medium; and comparing said nucleic acid sequence to at least one other nucleic acid sequence to identify identity.

In another aspect of the invention there is provided a method for performing sequence identification, said method comprising the steps of providing one or more of the following polymorphism containing nucleic acid sequences:
the nucleic acid disclosed in EMBL Accession Number X51602 with A at position 1953 according to the nucleotide positioning therein;
the nucleic acid sequence disclosed in EMBL Accession Number X51602 with T at position 3453 according to the nucleotide positioning therein;
the nucleic acid sequence disclosed in EMBL Accession Number X51602 with C at position 3888 according to the nucleotide positioning therein;
the nucleic acid sequence disclosed in EMBL Accession Number D64016 with T at position 519 according to the nucleotide positioning therein;
the nucleic acid sequence disclosed in EMBL Accession Number D64016 with T at position 786 according to the nucleotide positioning therein;
the nucleic acid sequence disclosed in EMBL Accession Number D64016 with T at position 1422 according to the nucleotide positioning therein;
the nucleic acid sequence disclosed in EMBL Accession Number D64016 with T at position 1429 according to the nucleotide positioning therein;
the nucleic acid sequence disclosed in SEQ ID No. 3 with G at position 454 according to the nucleotide positioning therein;
the nucleic acid sequence disclosed in SEQ ID No. 3 with A at position 454 according to the nucleotide positioning therein;
the nucleic acid sequence disclosed in SEQ ID No. 5 with T at position 696 according to the nucleotide positioning therein;
the nucleic acid sequence disclosed in SEQ ID No. 5 with C at position 696 according to the nucleotide positioning therein;
or a complementary strand thereof or a fragment thereof of at least 17 bases comprising at least one of the polymorphisms, and comparing said nucleic acid sequence to at least one other nucleic acid or polypeptide sequence to determine identity.

The invention will now be illustrated but not limited by reference to the following Examples. All temperatures are in degrees Celsius.

In the Examples below, unless otherwise stated, the following methodology and materials have been applied.

AMPLITAQ‰, available from Perkin-Elmer Cetus, is used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989).

Electropherograms were obtained in a standard manner: data was collected by ABI377 data collection software and the wave form generated by ABI Prism sequencing analysis (2.1.2).

### EXAMPLES

### Example 1 - Identification of Polymorphisms

### A. Methods

The polymorphism scan of the coding region of the flt-1 gene was performed on cDNA generated from total RNA isolated from lymphoblastoid cell lines derived from unrelated individuals (Coriel Institute). The polymorphism scan of the 3' UTR and promoter regions was performed on genomic DNA.

### DNA Preparation

DNA was prepared from frozen blood samples collected in EDTA following protocol I (Molecular Cloning: A Laboratory Manual, p392, Sambrook, Fritsch and Maniatis, 2^{nd} Edition, Cold Spring Harbor Press, 1989) with the following modifications. The thawed blood was diluted in an equal volume of standard saline citrate instead of phosphate buffered saline to remove lysed red blood cells. Samples were extracted with phenol, then phenol/chloroform and then chloroform rather than with three phenol extractions. The DNA was dissolved in deionised water. Total RNA was isolated from lymphoblastoid cells and converted to cDNA by standard protocols (Current Protocols in Molecular Biology FM Ausubel et al Volume 1 John Wiley 1998)

### Template Preparation

Templates were prepared by PCR using the oligonucleotide primers and annealing temperatures set out below. The extension temperature was 72° and denaturation temperature 94°. Generally 50 ng of genomic DNA or cDNA was used in each reaction and subjected to 35 cycles of PCR. In some cases, two rounds of amplification were required to generate products from cDNA, the oligonucleotides used primary and secondary amplification are listed.

### Dye Primer Sequencing

Dye-primer sequencing using M13 forward and reverse primers was as described in the ABI protocol P/N 402114 for the ABI Prism™ dye primer cycle sequencing core kit with "AmpliTaq FS"™ DNA polymerase, modified in that the annealing temperature was 45° and DMSO was added to the cycle sequencing mix to a final concentration of 5 %.

The extension reactions for each base were pooled, ethanol/sodium acetate precipitated, washed and resuspended in formamide loading buffer.

4.25 % Acrylamide gels were run on an automated sequencer (ABI 377, Applied Biosystems).

### B. Results

### Primer design

### 1. Primer locations for scan of Coding region and 3'UTR

All locations in this section refer to EMBL Accession X51602

### EMBL Accession Number X51602, 7680 bp

### 5' UTR (1-249), Coding (250-4266), 3'UTR (4267-7680)

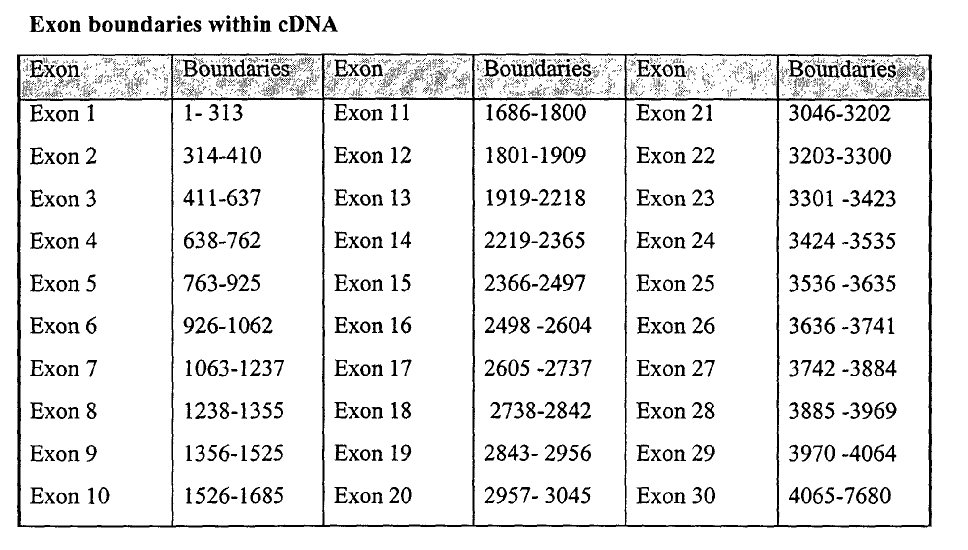

### Products requiring two stage amplification from c DNA

| **Primary product** | | | | |
|---|---|---|---|---|
| **Product** | **Forward Primer** | **Reverse Primer** | **Temp °C** | **Time** |
| 1777-3946 | 1777-1804 | 3919-3946 | 55 | 3 min |

| **Secondary Products (Primary product diluted 1000 x)** | | | | |
|---|---|---|---|---|
| **Product** | **Forward Primer** | **Reverse Primer** | **Temp °C** | **Time** |
| a. 1854-2435 | 1854-1877 | 2412-2435 | 58 | 90 sec |
| b. 2288-2879 | 2288-2311 | 2857-2879 | 58 | 90 sec |
| c. 2723-3310 | 2723-2746 | 3288-3310 | 58 | 90 sec |
| d. 3157-3748 | 3157-3180 | 3725-3748 | 58 | 90 sec |

| **Products amplified directly from cDNA** | | | | |
|---|---|---|---|---|
| **Product** | **Forward Primer** | **Reverse Primer** | **Temp °C** | **Time** |
| e. 293-696 | 292-313 | 673-696 | 55 | 90 sec |
| f. 564-1133 | 564-587 | 1110-1133 | 55 | 90 sec |
| g. 1031-1626 | 1031-1054 | 1603-1626 | 55 | 90 sec |
| h. 1491-2046 | 1491-1514 | 2023-2046 | 55 | 90 sec |
| i. 3662-4249 | 3662-3682 | 4226-4249 | 55 | 90 sec |

| **Products amplified from genomic DNA** | | | | |
|---|---|---|---|---|
| **Product** | **Forward Primer** | **Reverse Primer** | **Temp °C** | **Time** |
| j. 4163-4744 | 4163-4182 | 4721-4744 | 55 | 90 sec |

### 2. Primer locations for scan of promoter, 5' UTR, exon 1

All locations in this section refer to EMBL Accession Number D64016

### EMBL Accession Number D64016, 1745 bp

### Promoter region, exon 1, intron 1

| **Product** | **Forward Primer** | **Reverse Primer** | **Temp °C** | **Time** |
|---|---|---|---|---|
| k. 14-479 | 14-34 | 456-479 | 55 | 90 sec |
| 1. 343-890 | 343-366 | 869-890 | 55 | 90 sec |
| m. 762-1251 | 762-781 | 1232-1251 | 55 | 90 sec |
| n. 1151-1694 | 1151-1172 | 1673-1694 | 55 | 90 sec |

For dye-primer sequencing these primers were modified to include the M13 forward and reverse primer sequences (ABI protocol P/N 402114, Applied Biosystems) at the 5' end of the forward and reverse oligonucleotides respectively.

### Novel Polymorphisms

### Novel Polymorphisms within coding region - numbering refers to EMBL Accession Number X51602

| **(1)** | | | |
|---|---|---|---|
| **Position** | **Polymorphism** | **Allele Frequency** | **No of Individuals** |
| 1953 | G/A | G 90% A 10% | 31 |

Polymorphism at position 1953 alters the third base of codon 568 (Threonine - ACG/ACA). It has been shown that single nucleotide polymorphisms can cause different structural folds of mRNA with potentially different biological functions (Shen et al 1999, *ibid*). The polymorphism can be detected by a diagnostic e RFLP since engineering of positions 1949,1950 creates a BsiWI recognition sequence (CGTACG). Polymorphism at position 1953 will modify the recognition sequence (CGTAC**G/A**).

### Diagnostic primer (positions 1919-1952 in X51602)

ATGGGTTTCATGTTAACTTGGAAAAAATGCGTAC

modified residues in bold underline

### Reverse primer (positions 2098-2125 in X51602)

CATTCATGATGGTAAGATTAAGAGTGAT

Amplification of genomic DNA with these primers will generate a PCR product of 206 bp. Digestion of a product from a wild type template with BsiWI (New England Biolabs) will give rise to products of 168bp and 38 bp. Digestion of a heterozygote product will generate products of 206 bp,168 bp and 38 bp. A product generated from a homozygote variant will not be digested by BsiWI. Products can be separated and visualised on agarose gels following standard procedures (i.e. Molecular Cloning: Sambrook et al., 1989, *ibid*).

| **(2)** | | | |
|---|---|---|---|
| **Position** | **Polymorphism** | **Allele Frequency** | **No of Individuals** |
| 3453 | C/T | C 70% T 30% | 23 |

Polymorphism at position 3453 alters the third base of codon 1068 (Proline - CCC/CCT). It has been shown that single nucleotide polymorphisms can cause different structural folds of mRNA with potentially different biological functions (Shen et al 1999, *ibid*). The polymorphism at position 3453 can be detected by a diagnostic e RFLP, since modification of positions 3455, 3456, 3457 creates a PstI recognition sequence (CTGCAG). Polymorphism at position 3453 will modify the recognition sequence (CTGC**A/T**G).

### Diagnostic primer (Reverse, positions 3487-3454 in X51602, equivalent to positions 330-297 in Seq ID No 3)

TCTTGGTTGCTGTAGATTTTGTCAAAGATAGCTGC

Modified residues in bold underline

### Forward primer (position 193-216 in Seq ID No 3)

ACCCCATGGACACTCGGGTTGAAT

Amplification of genomic DNA with these primers will generate a PCR product of 137 bp. A product generated from a wild type template will not be digested by PstI (New England Biolabs). Digestion of a heterozygote product will give rise to products of 137 bp, 102 bp and 35 bp, digestion of a homozygous product will give rise to products of 102 bp and 35 bp. Products can be separated and visualised on agarose gels following standard procedures (i.e. Molecular Cloning: Sambrook et al., 1989, *ibid*).

| **(3)** | | | |
|---|---|---|---|
| **Position** | **Polymorphism** | **Allele Frequency** | **No of Individuals** |
| 3888 | T/C | T 74% C 26% | 23 |

Polymorphism at position 3888 alters the third base of codon 1213 (Tyrosine - TAT/TAC). It has been shown that single nucleotide polymorphisms can cause different structural folds of mRNA with potentially different biological functions (Shen et al 1999, *ibid*). Polymorphism at position 3888 creates a SnalB recognition sequence (TA**C**GTA).

### Forward primer (Positions 362-385 in Seq ID No 5)

CCTCAACCCTACAGAATGTGAATTG

### Reverse primer (Positions 828-804 in Seq ID No 5)

CAGCTAGGTCTAGTTGTCAGTCCTC

Amplification of genomic DNA with these primers will generate a PCR product of 467 bp. A product generated from a wild type template will not be digested by SnalB (New England Biolabs). Digestion of a heterozygote product will give rise to products of 467 bp, 245 bp and 222 bp, digestion of a homozygous variant product will generate products of 245 bp and 222 bp. Products can be separated and visualised on agarose gels following standard procedures (i.e. Molecular Cloning: Sambrook et al., 1989, *ibid*).

### Novel polymorphisms within promoter and 5'UTR -numbering refers to EMBL Accession Number D64016

| **(4)** | | | |
|---|---|---|---|
| **Position** | **Polymorphism** | **Allele Frequency** | **No of Individuals** |
| 519 | C/T | C 97% T 3% | 34 |

The polymorphism at position 519 can be detected by a diagnostic e RFLP, since modification of position 516 creates a potential SphI recognition sequence (GCATGC). Polymorphism at position 519 will modify the recognition sequence (GCA**C/T**GC).

### Diagnostic primer (Positions 485-518 in D64016)

GGGTGCATCAATGCGGCCGAAAAAGACACGGCA

Modified residues in bold underline

### Constant primer (Positions 724-741 in D64016)

GTGTTCTTGGCACGGAGG

Amplification of genomic DNA with these primers will generate a PCR product of 256 bp. A product generated from a wild type template will not be digested by SphI (New England Biolabs). Digestion of a heterozygote product will generate products of 256 bp, 221 bp and 35 bp, digestion of a homozygote variant product will generate products of 221 bp and 35 bp. Products can be separated and visualised on agarose gels following standard procedures (i.e. Molecular Cloning: Sambrook et al., 1989, *ibid*).

| **(5)** | | | |
|---|---|---|---|
| **Position** | **Polymorphism** | **Allele Frequency** | **No of Individuals** |
| 786 | C/T | C 98% T 2% | 50 |

The polymorphism at position 786 can be detected by a diagnostic e RFLP, since modification of position 781,782 creates a NarI recognition sequence (GGCGCC). Polymorphism at position 786 will modify the recognition sequence (GGCGC**C/T**).

### Diagnostic primer (Positions 751-785 in D64016)

GGCGCGGCCAGCTTCCCTTGGATCGGACTTGGCGC

Modified residues in bold underline

### Constant primer (Positions 869-890 in D64016)

Amplification of genomic DNA with these products will generate a PCR product of 139 bp. Digestion of a product from a wild type template with NarI (New England Biolabs) will generate products of 105 bp and 34 bp. Digestion of a heterozygote product will generate products of 139 bp, 105 bp and 34 bp. The homozygous variant product will not be digested by NarI. Products can be separated and visualised on agarose gels following standard procedures (i.e. Molecular Cloning: Sambrook et al., 1989, *ibid*).

| **(6)** | | | |
|---|---|---|---|
| **Position** | **Polymorphism** | **Allele Frequency** | **No of Individuals** |
| 1422 | C/T | C 98% T 2% | 25 |

Polymorphism at position 1422 alters an EagI recognition sequence (CGGC/TCG).

### Forward Primer (Positions 1251-1272 in D64016)

### Reverse primer (Positions 1673-1694 in D64016)

Amplification of genomic DNA with these primers generates a PCR product of 443 bp. Digestion of product from a wild type template with Eag I (New England Biolabs) will generate products of 271 bp and 143 bp. Digestion of a heterozygote product will generate products of 443 bp, 271 bp and 143 bp. The homozygous variant product will not be cleaved by Eag I. Products can be separated and visualised on agarose gels following standard procedures (i.e. Molecular Cloning: Sambrook et al., 1989, *ibid*).

| **(7)** | | | |
|---|---|---|---|
| **Position** | **Polymorphism** | **Allele Frequency** | **No of Individuals** |
| 1429 | G/T | G 76% T 24% | 25 |

The polymorphism at position 1429 can be detected by a diagnostic e RFLP, since modification of position 1431,1432 creates a Hinc II recognition sequence (GTTGAC). Polymorphism at position 1429 will modify the recognition sequence (**G/T**TTGAC).

### Diagnostic primer (Reverse, positions 1430-1463 in D64016)

CTGCTCGCCCGGTGCCCGCGCTCCCCGCGGTTAA

Modified bases in bold underline

### Constant primer (Forward, positions 1251-1272 in D64016)

Amplification of genomic DNA with these primers will generate a PCR product of 212 bp. Digestion of product from a wild type template with Hinc II (New England Biolabs) will generate products of 178 bp and 34 bp, digestion of a heterozygote product will give rise to products of 212 bp, 178 bp and 34 bp. A homozygote variant product will not be digested by Hinc II. Products can be separated and visualised on agarose gels following standard procedures (i.e. Molecular Cloning: Sambrook et al., 1989, *ibid*).

### Novel polymorphism identified in intron 24

### Primer locations for scan of intron 24, All locations in this section refer to Seq ID No 3.

| **Product** | **Forward Primer** | **Reverse Primer** | **Temp** | **Time** |
|---|---|---|---|---|
| 193-538 | 193-216 | 538-515 | 55°C | 90 sec |

| **Position** | **Polymorphism** | **Allele Frequency** | **No of Individuals** | |
|---|---|---|---|---|
| 454 | G/A | G 76% A 24% | 23 | |

### Novel polymorphism identified in intron 28

### Primer locations for scan of intron 28, All locations in this section refer to Seq ID No 5.

| **Product** | **Forward Primer** | **Reverse Primer** | **Temp** | **Time** |
|---|---|---|---|---|
| 362-828 | 362-385 | 828-804 | 55°C | 90 sec |

| **Position** | **Polymorphism** | **Allele Frequency** | **No of Individuals** | |
|---|---|---|---|---|
| 696 | T/C | T 76% C 24% | 23 | |

### Novel genomic sequence flanking exons within the human flt-1 gene

Two overlapping BAC clones were isolated - 51L6 (5') and 87P12 (3')

| **Sequencing Primers (positions refer to Accession X 51602)** | |
|---|---|
| **Exon 17 (BAC clone 87P12)** | |
| Forward | 2641-2664 |
| Reverse | 2664-2641 |
| | |
| **Exon 21 (BAC clone 87P12)** | |
| Forward | 1357-1380 |
| Reverse | 1380-1357 |
| | |
| **Exon 24 (BAC clone 87P12)** | |
| Forward | 3452-3478 |
| Reverse | 3529-3506 |
| | |
| **Exon 27 (BAC clone 87P12)** | |
| Forward | 3785-3811 |
| Reverse | 3811-3785 |
| | |
| **Exon 28 (BAC Clone 87P12)** | |
| Forward | 3918-3946 |
| Reverse | 3946-3918 |

## Claims

1. A method for the diagnosis of one or more single nucleotide polymorphism(s) in flt-1 gene in a human, which method comprises determining the sequence of the nucleic acid of the human at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), and determining the status of the human by reference to polymorphism in the flt-1 gene.

2. A method according to claim 1 in which the single nucleotide polymorphism at position 1953 (according to the position in EMBL accession number X51602) is the presence of G and/or A; and/or at position 3453 (according to the position in EMBL accession number X51602) is the presence of C and/or T; and/or at position 3888 (according to the position in EMBL accession number X51602) is the presence of T and/or C; and/or at position 519 (according to the position in EMBL accession number D64016) is the presence of C and/or T; and/or at position 786 (according to the position in EMBL accession number D64016) is the presence of C and/or T; and/or at position 1422 (according to the position in EMBL accession number D64016) is the presence of C and/or T; and/or at position 1429 (according to the position in EMBL accession number D64016) is the presence of G and/or T; and/or at position 454 (according to the position in SEQ ID No. 3) is the presence of G and/or A; and/or at position 696 (according to the position in SEQ ID No. 5) is the presence of T and/or C.

3. A method as claimed in claim 1 or 2, wherein the nucleic acid region containing the potential single nucleotide polymorphism is amplified by polymerase chain reaction prior to determining the sequence.

4. A method as claimed in any of claims 1 - 3, wherein the presence or absence of the single nucleotide polymorphism is detected by reference to the loss or gain of, optionally engineered, sites recognised by restriction enzymes.

5. A method according to claim 1 or claim 2, in which the sequence is determined by a method selected from ARMS-allele specific amplification, allele specific hybridisation, oligonucleotide ligation assay and restriction fragment length polymorphism (RFLP).

6. A method as claimed in any of the preceding claims for use in assessing the predisposition and/or susceptibility of an individual to diseases mediated by an flt-1 ligand.

7. A method for the diagnosis of flt-1 ligand-mediated disease, which method comprises:
i) obtaining sample nucleic acid from an individual;
ii) detecting the presence or absence of a variant nucleotide at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), in the flt-1 gene; and,
iii) determining the status of the individual by reference to polymorphism in the flt-1 gene.

8. An isolated nucleic acid comprising at least 17 consecutive bases of flt-1 gene said nucleic acid comprising one or more of the following polymorphic alleles: A at position 1953 (according to X51602), T at position 3453 (according to X51602), C at position 3888 (according to X51602), T at position 519 (according to D64016), T at position 786 (according to D64016), T at position 1422 (according to D64016), T at position 1429 (according to D64016), A at position 454 (according to SEQ ID No. 3) and C at position 696 (according to SEQ ID No. 5), or a complementary strand thereof.

9. An allele specific primer or probe capable of detecting an flt-1 gene polymorphism at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5).

10. A primer as claimed in claim 9 which is an allele specific primer adapted for use in ARMS.

11. An allele specific nucleotide probe as claimed in claim 9 which comprises the sequence disclosed in any one of SEQ ID Nos: 6 - 14, or a sequence complementary thereto.

12. A diagnostic kit comprising one or more diagnostic primer(s) and/or allele-specific oligonucleotide probes(s) as defined in claims 9, 10 or 11.

13. Use of an flt-1 ligand antagonist drug in the preparation of a medicament for treating a VEGF-mediated disease in a human diagnosed as having a single nucleotide polymorphism at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), in the flt-1 gene.

14. A pharmaceutical pack comprising an flt-1 ligand antagonist drug and instructions for administration of the drug to humans diagnostically tested for a single nucleotide polymorphism at one or more of positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5), in the flt-1 gene.

15. An isolated nucleic acid sequence comprising the sequence selected from the group consisting of:
(i) the nucleotide sequence from positions 1-482 of SEQ ID No. 1;
(ii) the nucleotide sequence from positions 616-1073 of SEQ ID No. 1;
(iii) the nucleotide sequence from positions 1-437 of SEQ ID No. 2;
(iv) the nucleotide sequence from positions 595-1024 of SEQ ID No. 2;
(v) the nucleotide sequence from positions 1123-1480 of SEQ ID No. 2;
(vi) the nucleotide sequence from positions 1-266 of SEQ ID No. 3;
(vii) the nucleotide sequence from positions 279-726 of SEQ ID No. 3;
(viii) the nucleotide sequence from positions 1-284 of SEQ ID No. 4;
(ix) the nucleotide sequence from positions 391-651 of SEQ ID No. 4;
(x) the nucleotide sequence from positions 795-1352 of SEQ ID No. 4;
(xi) the nucleotide sequence from positions 1-579 of SEQ ID No. 5;
(xii) the nucleotide sequence from positions 665-1256 of SEQ ID No. 5;
(xiii) a nucleotide sequence having at least 80%, preferably at least 90%, sequence identity to a sequences (i) - (xii);
(xiv) an isolated fragment of (i) - (xiii); and
(xv) a nucleotide sequence fully complementary to (i) - (xiv).

16. A computer readable medium having stored thereon a nucleic acid sequence comprising at least 20 consecutive bases of the flt-1 gene sequence, which sequence includes at least one of the polymorphisms at positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5).

17. A computer readable medium having stored thereon a nucleic acid comprising any of the intron sequences disclosed in any of SEQ ID Nos. 1 - 5.

18. A method for performing sequence identification, said method comprising the steps of providing a nucleic acid sequence comprising at least 20 consecutive bases of the flt-1 gene sequence, which sequence includes at least one of the polymorphisms at positions: 1953, 3453, 3888 (each according to the position in EMBL accession number X51602), 519, 786, 1422, 1429 (each according to the position in EMBL accession number D64016), 454 (according to SEQ ID No. 3) and 696 (according to SEQ ID No. 5) in a computer readable medium; and comparing said nucleic acid sequence to at least one other nucleic acid sequence to identify identity.


SEQUENCE LISTING

<110> SMITH, JOHN C
      ASTRAZENECA AB

<120> DIAGNOSTIC METHOD

<130> LDSG/70675

<140>
<141>

<150> GB0004232.5
<151> 2000-02-24

<160> 24

<170> PatentIn Ver. 2.1

<210> 1
<211> 1073
<212> DNA
<213> Human

<400> 1
   gggtttactt tgccacttct tgcttttcct atatatgtag aaaagccaca gtgcgcccca 60
   ctgttggccc atatgtaata tatattcctg cttatacaag atggccatgg gaagttattt 120
   ttagtcattg tttggaatga ctttataaaa atgctttgca ttttttagca agaccatcat 180
   ataattgttt aagatcaagt acaacacata aggtcactgg agaatttgag tgcatgttat 240
   ccaagatagg atggtagagc tcacattaca gaaatgtagt gtgggaatag taaggagtcg 300
   tttaatagaa attgcacacc taagtgtgat gagtgtatgt gaatgtggag aagtactttc 360
   tgcacctggc cacacagttt caaccaaatg atcccnaaat aaaacagtgg atgttaacgg 420
   aatatctagg atttgtaaag ttgttttctt ctcgatgact ttgagatctc tttatttctc 480
   agtcttcttc tgaaataaag actgactacc tatcaattat aatggaccca gatgaagttc 540
   ctttggatga gcagtgtgag cggctccctt atgatgccag caagtgggag tttgcccggg 600
   agagacttaa actgggtaag atatttgttc aacagattca taaacctata ctgagcacat 660
   attacatgaa aaacactgtg ctttgagaga tgcgaaagta aactagacct gggattctac 720
   cctccagctg ctcacagact agcaagggag atggacacaa aagtaaataa ttccaatgca 780
   atgctcagat aacagtacaa ggtgacacgc agcacctgtt tgttcttgca acagttatta 840
   ggcaccttct ctgagcagca gacactggtc taagccctgg agacacaaag gtgcttgcat 900
   ctcttccctc aaagggctca gtctggagat aggtgcaaaa gtggtaagtg aaggggggcg 960
   gagagagagg cattacaagt acacgcacgc ttcataatga aactgttgag ggattagaaa 1020
   tatgtgatcc agaacataat tgagggtggc aaggaacagt gaaatcaaca ttc 1073

<210> 2

<211> 1480

<212> DNA

<213> Human

<400> 2

cactgtgccc ggccagcttt gctatttatt agctgcatgt gaatttgatt actttacttc 60

tctgaacctg tttctccatg tataaataag aactacttcg taaaattgtt ggaaacacta 120

aacaagaaat gnacctaaag cttttaatat accagctcac acagagtaag cattcagtaa 180

atacccacca ctcttaattt ttttttttta tctgatctaa gatgctgtct agaagcccag 240

gcaagagcac aatagactct gcaactccag aggtagtcag gctcctggac accgtagggc 300

ccctgtgcta gttcacgatc cattttgaga agtgaaacgc tctcatttct catcaggcna 360

ttgccagttg agggactggt ttcccnctgc tgtgctggag ctccttttca cctgggtcct 420

tttcggtctc ttcaaaggat gcagcactac acatggagcc taagaaagaa aaaatggagc 480

caggcctgga acaaggcaag aaaccaagac tagatagcgt caccagcagc gaaagctttg 540

cgagctccgg ctttcaggaa gataaaagtc tgagtgatgt tgaggaagag gagggtaggt 600

attaattcct tcctgtccta cgcgctgaga tatttttaca acatactatg catctctgaa 660

atttttttct tatttatcac tctaataaac atccgtggga gactcgaatg gtaatgtcct 720

gaggagataa gatttgaatt aagataattt acagagttac taattttgac agggaactgt 780

accgttttct cccctcaggg attttcatct taatggatca tccccctgcc cccatgcttg 840

gataaagtgg gctggaggcc tggaaaaatc tctggtgttc atgttgaaac tcaaatactc 900

ttaaaaatga actctgatct acttgttggt ttgttttatg ttttgctaac attgttccaa 960

taaactggga tttggtggga taacaagagc cattacaaac agttacggtt ctaatgcttt 1020

ccagattctg acggtttcta caaggagccc atcactatgg aagatctgat ttcttacagt 1080

tttcaagtgg ccagaggcat ggagttcctg tcttccagaa aggtcagtct tgctgtttac 1140

tgtttttctt ctctgccagg gctggacaca cacctttgct ataaattcat ttttcctagt 1200

atttgctgat acctatgttc ttaaatgtag aacaaacacc actgcaagtg ccttaatttg 1260

ccttgatatg aggagttttg agaatgagga gtcatggata ccagtggata gaacttaatt 1320

ctggggaaaa ctcacaggtt tcagactaga caaacctggc atcggctctc cacagtatcc 1380

tctggcatat tttcaaatct ggcccaaatc tcagaagaca tgacttcata ggagagctac 1440

tattaatata gccatatagg gccctcccac aaaactgcag 1480

<210> 3

<211> 726

<212> DNA

<213> Human

<400> 3

cagagctatg cagataagga catgctgaac acatcagagg ggcttactga acatatacng 60
ccttcatggg actcagtata gcactctagc tccctctttt agcgtaacac tgcatactat 120
ggtgttctct atgttaggaa accagagctg ctctcggaaa tgatttatag gccgtatgtt 180
atctgggagg tgaccccatg gacactcggg ttgaatgtgc tttgttttca tgcccttctg 240
ctcaaggccc ccttgccctc ttctagactc gacttcctct gaaatggatg gctcctgaat 300
ctatctttga caaaatctac agcaccaaga gcgacgtgtg gtcttacgga gtattgctgt 360
gggaaatctt ctccttaggt aaatttggga gaaggaagaa atcaaacagc ccagaaataa 420
atgtctgcat cttctgctga atgtcctttg gttggacagc ctttagatta gaacctactg 480
taacaaaaaa ctcttaaagt gtaatgggcc catgtagact ctcagatgag taatggcgta 540
cgcatgtctg ccctctactg taaaagggct ttatatgatc atgaacaagg tcagaacaag 600
gtcatgtaaa agggctttat acgatcatga acaagggtat aaagtctgaa gcaaagtact 660
ttttctgtac tttgccaatt ctgccttttc aattcctcaa cacccacacc tctaatgccc 720
ttaccg 726

<210> 4
<211> 1352
<212> DNA
<213> Human

<400> 4
ctgcagaggc cacaggcaca acaaagaacc tgggtatcca tgagctctgg tgggttggtt 60
agtctgcctt ggtagacgtg ttttccactg accacaggac ctggcccaga cagcctttta 120
agtgctggtg ctataaaccc aaacctaaaa atgaagcagg gtcacatagt acagaaagct 180
tgggctttat gcggatgatg acagccctcc ctttgtagta cgtaaggcaa tgcataggat 240
gatcactgct ctccaactat ttctgttgct gttttcccca ccagctatca gatcatgctg 300
gactgctggc acagagaccc aaaagaaagg ccaagatttg cagaacttgt ggaaaaacta 360
ggtgatttgc ttcaagcaaa tgtacaacag gtaaaactaa atttatctac atcaaaatgc 420
ctttgaatgt acgtcagggg ggcattttat ttgttttttt tttaagagct attaatataa 480
tagctgagat cagaagttta aaaaaagggt gtgtgtgtgt gtatacagaa ttatcttctc 540
aaaacacaac caagattgtg gcaaatgaca tagtcaaagt tgacataatg gttcatagaa 600
attgttgaag tcagaattgg tgcaacgaga gctctacctt tggtatttta ggatggtaaa 660
gactacatcc caatcaatgc catactgaca ggaaatagtg ggtttacata ctcaactcct 720
gccttctctg aggacttctt caaggaaagt atttcagctc cgaagtttaa ttcaggaagc 780
tctgatgatg tcaggtaaga tttctttctc aaactttata tcacagaatt ttccaacaaa 840
aaaaagaaag aaagaaagac gaaagagaaa gaaagacnga aagagagaaa gaaagagaga 900
aagaaagaaa gagagaaaga aagaaagaaa gattatgttg atcaccaccc atatgcccat 960
cccctaaatt caactgttaa cattttgccc tattttgtct attatactct ctatgattgt 1020
gtttgttacg gatttttctt tttgccaaac catttaaaag gaggcttaaa gcataatagc 1080
actttactcc taaatacttt agtatacatt ttgtaagaag gctattgttg ctgggcacag 1140
tggctcgtgc ctgtaatcgc agcactttgg gagactgagg tgggaggatc acttgagcct 1200
aggagttcaa aatctgcctc ggcaacatag agagacctca tcttactaaa aatttaaaaa 1260

ttagccgggt gtggtggtgg gcacctgtag tcccagctac tcaggaggct gaggttggag 1320
gatcacttga gcccaggaga tggaggctgc ag 1352

<210> 5
<211> 1256
<212> DNA
<213> Human

<400> 5
agtggatgtc tccaatagtc tttcctaata catcatcaac aaaaggtcag taggtagtta 60
tagagacatc atacaacact acccaattct tcccaatctg taatcacaca cacacacaaa 120
atacaagcct ggcactagca ctcgattatg ccattaaata atatttagcc gtgtagccat 180
gccaggtcac tttgccacct cacatccttt tcagagcacc tgataaagtc ataccacttc 240
cctgcacatc atttctctcc tgtgccattg ggcactcaga cgagatgatg cctccagtct 300
ctcctacgtc tggcattctc tgatttcaca acggaccaga gtaggtccct ctgggagttt 360
cctcaaccct acagaatgtg aattgacaac cacgggaggc agtggcaatg ctgtcaggat 420
tcccaggggt cacggcgggg agatcggggc ctcaggagtt aggtgattcc tgttggtgtg 480
ttggttcatc ttagctggga tatggtgcct gtggtctcct gactcattag agctggatgc 540
cttttcctgt cttgataatt ctttctgttt cttcattaga tatgtaaatg ctttcaagtt 600
catgagcctg gaaagaatca aaacctttga agaactttta ccgaatgcca cctccatgtt 660
tgatgtaagt cgtgaagtta aggtacctag tgcactccga tagacccctt cttcagatcc 720
cttccaaaca ccaacgccag taatgtagta gttcttggtc agtgagggtc tggattcagg 780
agtggctgaa atgacagtgt ggggaggact gacaactaga cctagctgtg cagaactaat 840
ttgaaagtag agttccatgc actcactcca ggacccaagt ccctgcgtgg taggaattta 900
gaccctgagg aaactccatt gtgtgtttct aagctgctta gctgtcagtg atgcagcttt 960
gctttcagag taacagagga actcccagct gtgtgggtga tgggctttgt gatgtaacag 1020
agagcgcgtt cctgcaagca gccttgaggc tgggaggggt ccacctaagc cttatgctcc 1080
tttcccctga ggttctacag attgaacagc tgtgttccta cccaatcaca atgggagaag 1140
ctaaccagta tagcctggca aacaagaggt cttccagctc ttctctctaa agccctgtga 1200
tgtggggttg aggggctaag gggaggagag gagcatgggc aggagcgata ctgcag 1256

<210> 6
<211> 31
<212> DNA
<213> Human

<400> 6
ggaaaaaatg ccgacrgaag gagaggacct g 31

<210> 7

<211> 31

<212> DNA

<213> Human

<400> 7

gaaatggatg gctccygaat ctatctttga c                                31

<210> 8

<211> 31

<212> DNA

<213> Human

<400> 8

tgatgatgtc agataygtaa atgctttcaa g                                31

<210> 9

<211> 31

<212> DNA

<213> Human

<400> 9

aaaaagacac ggacaygctc ccctgggacc t                                31

<210> 10

<211> 31

<212> DNA

<213> Human

<400> 10

gatcggactt tccgcyccta gggccaggcg g                                31

<210> 11

<211> 31

<212> DNA

<213> Human

<400> 11

gacggactct ggcggycggg tctttggccg c                                    31

<210> 12
<211> 31
<212> DNA
<213> Human

<400> 12
tctggcggcc gggtckttgg ccgcggggag c                                    31

<210> 13
<211> 31
<212> DNA
<213> Human

<400> 13
gaatgtcctt tggttrgaca gcctttagat t                                    31

<210> 14
<211> 31
<212> DNA
<213> Human

<400> 14
aggtacctag tgcacyccga tagacccctt c                                    31

<210> 15
<211> 34
<212> DNA
<213> Human

<400> 15
atgggtttca tgttaacttg gaaaaaatgc gtac                                 34

<210> 16
<211> 28
<212> DNA

<213> Human

<400> 16
cattcatgat ggtaagatta agagtgat                                          28

<210> 17
<211> 35
<212> DNA
<213> Human

<400> 17
tcttggttgc tgtagatttt gtcaaagata gctgc                                  35

<210> 18
<211> 24
<212> DNA
<213> Human

<400> 18
accccatgga cactcgggtt gaat                                              24

<210> 19
<211> 25
<212> DNA
<213> Human

<400> 19
   cctcaaccct acagaatgtg aattg                                            25

<210> 20
<211> 25
<212> DNA
<213> Human

<400> 20
cagctaggtc tagttgtcag tcctc                                             25

<210> 21

<211> 33

<212> DNA

<213> Human

<400> 21

gggtgcatca atgcggccga aaaagacacg gca                                    33

<210> 22

<211> 18

<212> DNA

<213> Human

<400> 22

gtgttcttgg cacggagg                                                     18

<210> 23

<211> 35

<212> DNA

<213> Human

<400> 23

ggcgcggcca gcttcccttg gatcggactt ggcgc                                  35

<210> 24

<211> 34

<212> DNA

<213> Human

<400> 24

ctgctcgccc ggtgcccgcg ctccccgcgg ttaa                                   34